# EUROPEAN PATENT SPECIFICATION

(11) **EP 3 508 568 B1**
(45) Date of publication and mention of the grant of the patent: **03.03.2021**
(21) Application number: 17846789.0
(22) Date of filing: 28.04.2017
(51) Int. Cl.: C12N 1/20, A61K 35/74, A23L 33/135, C12R 1/01

(54) **LEUCONOSTOC HOLZAPFELII STRAIN HAVING HAIR LOSS PREVENTION, HAIR GROWTH PROMOTION OR SEXUAL FUNCTION IMPROVEMENT EFFECTS, AND COMPOSITION COMPRISING SAME**
LEUCONOSTOC-HOLZAPFELII-STAMM MIT HAARAUSFALLPRÄVENTIONS-, HAARWACHSTUMFÖRDERUNGS- ODER SEXUALFUNKTIONSVERBESSERUNGSEFFEKTEN UND ZUSAMMENSETZUNG DAMIT
SOUCHE DE LEUCONOSTOC HOLZAPFELII AYANT POUR EFFETS DE PRÉVENIR LA PERTE DES CHEVEUX, DE FAVORISER LA POUSSE DES CHEVEUX OU D'AMÉLIORER LA FONCTION SEXUELLE, ET COMPOSITION LA COMPRENANT

(30) Priority: 30.08.2016 KR 20160111042
(43) Date of publication of application: 10.07.2019
(73) Proprietor: Coenbio Co., Ltd., Seongnam-si, Gyeonggi-do 13207 (KR)
(72) Inventor: YUM, Kyu Jin, Anyang-si Gyeonggi-do 14106 (KR)
(74) Representative: Cabinet Beau de Loménie
(86) International application number: PCT/KR2017/004537
(87) International publication number: WO 2018/043864

(56) References cited:
- EP-A1- 1 097 700
- WO-A1-2013/002439
- JP-A- 2007 070 305
- KR-A- 20130 139 806
- KR-A- 20150 065 170
- KR-A- 20150 104 541
- KR-A- 20160 030 605
- KR-B1- 100 942 794
- K. DE BRUYNE ET AL: "Leuconostoc holzapfelii sp. nov., isolated from Ethiopian coffee fermentation and assessment of sequence analysis of housekeeping genes for delineation of Leuconostoc species", INTERNATIONAL JOURNAL OF SYSTEMATIC AND EVOLUTIONARY MICROBIOLOGY, vol. 57, no. 12, 1 December 2007 (2007-12-01), pages 2952-2959, XP055674474, GB ISSN: 1466-5026, DOI: 10.1099/ijs.0.65292-0

## Description

### [Technical Field]

The present invention relates to a novel strain capable of preventing hair loss, promoting hair growth or improving sexual function, and a composition including the strain.

### [Background Art]

Lactic acid bacteria ferment milk to produce lactic acid. Lactic acid bacteria inhibit the growth of intestinal harmful bacteria and promote the proliferation of beneficial bacteria to balance the intestinal environment, activating self-healing in the human body. In addition, lactic acid bacteria relieve constipation, improve gastrointestinal function, activate innate immunity, have anticancer effects, improve fatigue resistance, reduce subcutaneous fat and cholesterol levels, are effective in dieting, relieve cutaneous diseases such as atopic dermatitis, are effective in preventing aging, and are good for relieving hangovers. Such efficacy of lactic acid bacteria has been demonstrated by many preceding studies.

With the development of modern society, the number of alopecia patients has increased rapidly. Particularly, the age of alopecia patients has dropped gradually. 60% of people who require hair care service are in their twenties and thirties. The number of patients suffering from alopecia is estimated to reach almost 10 million, causing serious socioeconomic problems.

Reports say that people with severe hair loss are more likely to experience psychological depression and anxiety, perceive high levels of stress, are faced with difficulty in their relationships with other people and the opposite sex, and fail to maintain good family relationships. Thus, hair loss has become a matter of concern in health science.

Although the exact cause of hair loss is clearly unknown, reports say that hair loss is possibly caused by genetic, hormonological and immunological abnormalities.

Minoxidil for transdermal application and Finasteride for oral administration, approved by the U.S. Food and Drug Administration (FDA) are being currently widely used as therapeutic agents for alopecia. However, these therapeutic agents are limited in their use because of their attendant side effects such as cardiovascular disorders, skin irritation, decreased sexual function, and severe teratogenesis.

Previous studies reported that galenic preparations can ameliorate alopecia, but the results are still unsatisfactory. In recent years, some studies and patents reported that the use of fermentation products of plant extracts and hot water-extracted herbal materials such as fleeceflower root by lactic acid bacteria belonging to the genus *Lactobacillus* can promote hair growth. However, most of the results were obtained from experimental rats and no research has been conducted on people who had suffered or were suffering hair loss.

### [Disclosure]

### [Technical Problem]

The present invention is aimed at providing a novel lactic acid bacterial strain that is effective in preventing hair loss, promoting hair growth or improving sexual function.

The present invention is aimed at providing a composition for preventing hair loss, promoting hair growth or improving sexual function that can be used safely without causing side effects.

### [Technical Solution]

One embodiment of the present invention provides a novel *Leuconostoc holzapfelii* strain Ceb-kc-003 (KCCM11830P). The strain is morphologically Gram-positive, has the sequence set forth in SEQ ID NO: 1, and has a homology of 99% with *Leuconostoc holzapfelii,* as analyzed by 16s rRNA partial sequencing.

<SEQ ID NO: 1>
>Ceb-kc-003
GCGAGTGGCGAACGGGTGAGTAACACGTGGATAACCTACCTCAAGGCTGGGATAACATTT
GGAAACAGATGCTAATACCGAATAAAACTTAGTATCGCATGATACCAGGTTAAAGGCGCT
ACGGCGTCACCTAGAGATGGATCCGCGGTGCATTAGTTAGTTGGTGGGGTAAAGGCTTACCA
AGACAATGATGCATAGCCGAGTTGAGAGACTGATCGGCCACATTGGGACTGAGACACGGCC
CAAACTCCTACGGGAGGCTGCAGTAGGGAATCTTCCACAATGGGCGCAAGCCTGATGGAGC
AACGCCGCGTGTGTGATGAAGGCTTTCGGGTCGTAAAGCACTGTTGTATGGGAAGAAATGCT
AGAATAGGGAATGATTTTAGTTTGACGGTACCATACCAGAAAGGGACGGCTAAATACGTGC
CAGCAGCCGCGGTAATACGTATGTCCCGAGCGTTATCCGGATTTATTGGGCGTAAAGCGAGC
GCAGACGGTTGATTAAGTCTGATGTGAAAGCCCGGAGCTCAACTCCGGAATGGCATTGGAA
ACTGGTTAACTTGAGTGTTGTAGAGGTAAGTGGAACTCCATGTGTAGCGGTGGAATGCGTAG
ATATATGGAAGAACACCAGTGGCGAAGGCGGCTTACTGGACAACAACTGACGTTGAGGCTC
GAAAGTGTGGGTAGCAAACAGGATTAGATACCCTGGTAGTCCACACCGTAAACGATGAATA
CTAGGTGTTAGGAGGTTTCCGCCTCTTAGTGCCGAAGCTAACGCATTAAGTATTCCGCCTGG
GGAGTACGACCGCAAGGTTGA

The newly isolated strain *Leuconostoc holzapfelii* Ceb-kc-003 was deposited with the Korean Culture Center of Microorganisms (120-861, Hongje-2ga-Gil, Seodaemun-Ku, Seoul, Korea) on April 11, 2016 and assigned accession number KCCM11830P. The strain was isolated from kimchi, a traditional Korean fermented food. The strain is effective in preventing hair loss, promoting hair growth, improving sexual function, lowering cholesterol levels, and/or decomposing fat.

Another embodiment of the present invention relates to a culture, concentrate or dried product of *Leuconostoc holzapfelii* Ceb-kc-003 (KCCM11830P).

The newly isolated strain of the present invention can be cultured by any known method for culturing *Bacillus* or *Leuconostoc* strains. A natural or synthetic medium can be used to culture the strain. Examples of carbon sources of the medium include, but are not limited to, glucose, sucrose, dextrin, glycerol, and starch. Examples of nitrogen sources of the medium include, but are not limited to, peptone, meat extract, yeast extract, dried yeast, soybean, ammonium salt, nitrate, and other organic and inorganic nitrogenous compounds. One or more inorganic salts may be added to the medium. Examples of such inorganic salts include, but are not limited to, magnesium, manganese, calcium, iron, and potassium salts. The medium may further include one or more compounds selected from amino acids, vitamins, nucleic acids, and compounds related thereto. The newly isolated strain may be cultured at a temperature of 20 to 40°C for 12 hours to 4 days. As an example, one or more galenic preparations, an extract thereof or a mixture thereof may be added to and cultured in the culture medium. Examples of the galenic preparations include cooked Rehmannia, licorice root, Szechuan lovage root, Eucommia bark, Chinese cinnamon bark, Chinese angelica root, sweetflag rhizome, fleeceflower root, leafy twig of arorvitae, ginger, arbor-vitae seed, fruit of Szechuan pepper, bugbane rhizome, and vitex fruit. The use of the culture further improves the effect of the newly isolated strain to prevent hair loss, promote hair growth or improve sexual function.

The culture of the newly isolated strain may be a culture broth containing the bacterial cells. The culture may also be obtained by removal of the supernatant from the culture broth or concentration of the culture broth. The composition of the culture may further include not only one or more typical ingredients necessary for the culture of *Bacillus* or *Leuconostoc* strains but also one or more ingredients exerting a synergistic effect on the growth of *Bacillus* or *Leuconostoc* strains. The composition of the culture can be readily determined by those skilled in the art.

The strain may be in a liquid or dry state. The strain can be dried by any suitable technique known in the art. Examples of such drying techniques include, but are not limited to, air drying, natural drying, spray drying, and freeze drying.

A further embodiment of the present invention relates to a composition including a newly isolated strain *Leuconostoc holzapfelii* Ceb-kc-003 (KCCM11830P) or a culture solution, concentrate or dried product thereof. The composition may be used as a pharmaceutical composition for preventing hair loss, promoting hair growth or improving sexual function. The composition is also effective in decomposing fat and/or lowering cholesterol levels. Alternatively, the composition may be used as a food, health food, health supplement food or health functional food composition. One embodiment of the pharmaceutical composition may be a quasi-drug or a pharmaceutical preparation.

As an example, the composition may further include one or more galenic ingredients that are effective in preventing hair loss or promoting hair growth or one or more pharmaceutical ingredients. Examples of the galenic ingredients include galenic preparations such as cooked Rehmannia, licorice root, Szechuan lovage root, Eucommia bark, Chinese cinnamon bark, Chinese angelica root, sweetflag rhizome, red fleeceflower root, leafy twig of arorvitae, Acanthopanax senticosus, pine leaf and flower, buckwheat, Anemarrhena rhizome, ginger, arbor-vitae seed, fruit of Szechuan pepper, bugbane rhizome, and vitex fruit, extracts thereof, and mixtures thereof. Examples of the pharmaceutical ingredients include Minoxidil or Finasteride.

As another example, the composition may further include a strain belonging to the genus *Lactobacillus, Lactococcus, Propionibacterium, Enterocuccus* or *Bifidobacterium.* Specifically, the composition may further include *Leuconostoc mesenteroides* (*e.g.,* KCCM11827P), *Lactobacillus sakei (e.g.,* KCCM11841P), a culture solution thereof or a mixture thereof. More specifically, the composition may further include *Leuconostoc mesenteroides* KCCM11827P, *Lactobacillus sakei* KCCM11841P, a culture solution thereof or a mixture thereof. The composition may further include collagen.

The composition of the present invention includes the *Leuconostoc holzapfelii* strain Ceb-kc-003 (KCCM11830P) at a concentration of 5 × 10⁴ to 5 × 10¹⁰ CFU/ml. Preferably, the composition of the present invention includes the *Leuconostoc holzapfelii* Ceb-kc-003 (KCCM11830P) at a concentration of 1 × 10⁶ to 1 × 10⁹ CFU/ml.

The composition may further include a pharmaceutically or cytologically acceptable carrier. In this case, the composition may be formulated with the carrier to provide a food or pharmaceutical drug.

As used herein, the term "pharmaceutically or cytologically acceptable carrier" refers to a carrier or diluent that causes no irritation to target organisms and does not deteriorate the biological activity and properties of the strain.

The composition may be formulated into various preparations for oral or parenteral administration. Preferably, the composition is formulated into a liquid oral solution.

When formulated into a liquid solution, the pharmaceutically or cytologically acceptable carrier may be selected from saline solution, sterilized water, buffered saline, albumin injection solution, dextrose solution, maltodextrin solution, glycerol, and mixtures thereof that are sterile and biocompatible. If necessary, one or more general additives, such as antioxidants, buffer solutions, and bacteriostatic agents may be added to the composition. The composition of the present invention may be formulated into liquid preparations (such as aqueous solutions, suspensions or emulsions), pills, capsules, granules, and tablets. In this case, the composition of the present invention may further include one or more additives selected from diluents, dispersants, surfactants, binders, and lubricants. A binding agent, an emulsifier or preservative may be further added to the composition to prevent the quality of the composition from deteriorating. The composition of the present invention may further include one or more additives selected from amino acids, vitamins, enzymes, flavoring agents, non-protein nitrogen compounds, silicates, buffers, extractants, and oligosaccharides. The *Leuconostoc holzapfelii* strain Ceb-kc-003 (KCCM11830P) may be administered in the form of a liquid. In this case, the *Leuconostoc holzapfelii* strain Ceb-kc-003 (KCCM11830P) at a concentration of 5 × 10⁴ to 5 × 10⁸ CFU/ml, preferably 1 × 10⁶ to 1 × 10⁸ CFU/ml, may be administered 1 to 4 times (30 ml to 100 ml each time) daily. As an example, the *Leuconostoc holzapfelii* strain Ceb-kc-003 (KCCM11830P) at a concentration of 1 × 10⁶ to 1 × 10⁸ CFU/ml may be administered 2 to 4 times (50 ml each time) daily. More specifically, the *Leuconostoc holzapfelii* strain Ceb-kc-003 (KCCM11830P) at a concentration of 1 × 10⁶ to 1 × 10⁸ CFU/ml may be administered once or twice (50 ml to 100 ml each time) daily. Even more specifically, the *Leuconostoc holzapfelii* strain Ceb-kc-003 (KCCM11830P) at a concentration of 1 × 10⁶ to 1 × 10⁸ CFU/ml may be administered in an amount of 50 ml to 100 ml once 30 minutes before breakfast and in an amount of 50 ml to 100 ml once 30 minutes before dinner or before bed. As another example, the daily dose of the *Leuconostoc holzapfelii* strain Ceb-kc-003 (KCCM11830P) may be from 1.5 × 10⁵ to 5 × 10¹⁰ CFU.

Examples of oral preparations including the *Leuconostoc holzapfelii* strain Ceb-kc-003 (KCCM11830P) of the present invention include tablets, troches, lozenges, water-soluble or oily suspensions, powders, granules, emulsions, hard or soft capsules, syrups, and elixirs. The composition of the present invention may be formulated into desired preparations such as tablets and capsules. In this case, the composition may include: a binder such as lactose, saccharose, sorbitol, mannitol, starch, amylopectin, cellulose or gelatin; an excipient such as dicalcium phosphate; a disintegrant such as corn starch or sweet potato starch; or a lubricant such as magnesium stearate, calcium stearate, sodium stearyl fumarate or polyethylene glycol wax. For capsule preparations, the composition may further include a liquid carrier such as a fatty oil.

The daily dose of the composition or the *Leuconostoc holzapfelii* strain Ceb-kc-003 (KCCM11830P) may vary depending on the weight, age, sex, health condition, diet, main symptoms to be treated, prevented or ameliorated, time and mode of administration, and severity of disease and may be in the range of about 0.0001 mg/kg to about 10 g/kg, specifically about 0.01 mg/kg to about 500 mg/kg. The composition or the *Leuconostoc holzapfelii* strain Ceb-kc-003 (KCCM11830P) may be administered or applied 1 to 6 times, for example, 1 to 4 times, a day.

The *Leuconostoc holzapfelii* strain Ceb-kc-003 (KCCM11830P) may be present in a liquid or dry state, preferably a liquid state, in the composition. The strain can be dried by any suitable technique known in the art. Examples of such drying techniques include, but are not limited to, air drying, natural drying, spray drying, and freeze drying. The *Leuconostoc holzapfelii* strain Ceb-kc-003 (KCCM11830P) may be used in the form of a powder. In this case, the strain may be added in an amount of 0.05 wt% (% by weight) to 80 wt%, for example, 0.1 wt% to 70 wt%, 0.1 wt% to 50 wt%, or 1 wt% to 30 wt%, based on the weight of the composition.

One embodiment of the present invention relates to a composition for preventing hair loss or promoting hair growth including the *Leuconostoc holzapfelii* strain Ceb-kc-003 (KCCM11830P), a culture solution thereof, a concentrate thereof or a dried product thereof. A further embodiment of the present invention relates to a method for treating hair loss or promoting hair growth including administering to a subject in need of such prevention or treatment a therapeutically or prophylactically effective amount of the *Leuconostoc holzapfelii* strain Ceb-kc-003 (KCCM11830P), a culture solution thereof, a concentrate thereof or a dried product thereof. The therapeutically or prophylactically effective amount may vary depending on the weight, age, sex, health condition, main symptoms to be treated, prevented or ameliorated, time and mode of administration, and severity of disease, as described above, and may be in the range of about 0.0001 mg/kg to about 10 g/kg, specifically about 0.01 mg/kg to about 500 mg/kg, a day. The *Leuconostoc holzapfelii* strain Ceb-kc-003 (KCCM11830P) may be administered or applied 1 to 6 times, for example, 1 to 4 times, a day. The *Leuconostoc holzapfelii* strain Ceb-kc-003 (KCCM11830P) at a concentration of 5 × 10⁴ to 5 × 10⁸ CFU/ml, preferably 1 × 10⁶ to 1 × 10⁸ CFU/ml, may be administered 1 to 4 times (30 ml to 100 ml each time) daily. As an example, the *Leuconostoc holzapfelii* strain Ceb-kc-003 (KCCM11830P) at a concentration of 1 × 10⁶ to 1 × 10⁸ CFU/ml may be administered 2 to 4 times (50 ml each time) daily. More specifically, the *Leuconostoc holzapfelii* strain Ceb-kc-003 (KCCM11830P) at a concentration of 1 × 10⁶ to 1 × 10⁸ CFU/ml may be administered once to twice (50 ml to 100 ml each time) daily. Even more specifically, the *Leuconostoc holzapfelii* strain Ceb-kc-003 (KCCM11830P) at a concentration of 1 × 10⁶ to 1 × 10⁸ CFU/ml may be administered in an amount of 50 ml to 100 ml once 30 minutes before breakfast and in an amount of 50 ml to 100 ml once 30 minutes before dinner or before bed.

One embodiment of the present invention relates to a composition for ameliorating sexual dysfunction including the *Leuconostoc holzapfelii* strain Ceb-kc-003 (KCCM11830P), a culture solution thereof, a concentrate thereof or a dried product thereof. The sexual dysfunction may be for examples, erectile dysfunction. A further embodiment of the present invention relates to a method for preventing or treating sexual dysfunction including administering to a subject in need of such prevention or treatment a therapeutically or prophylactically effective amount of the *Leuconostoc holzapfelii* strain Ceb-kc-003 (KCCM11830P), a culture solution thereof, a concentrate thereof or a dried product thereof. The therapeutically or prophylactically effective amount is the same as that described above.

One embodiment of the present invention relates to a composition for reducing cholesterol levels including the *Leuconostoc holzapfelii* strain Ceb-kc-003 (KCCM11830P), a culture solution thereof, a concentrate thereof or a dried product thereof. A further embodiment of the present invention relates to a method for preventing or treating hypercholesterolemia including administering to a subject in need of such prevention or treatment a therapeutically or prophylactically effective amount of the *Leuconostoc holzapfelii* strain Ceb-kc-003 (KCCM11830P), a culture solution thereof, a concentrate thereof or a dried product thereof. The therapeutically or prophylactically effective amount is the same as that described above.

One embodiment of the present invention relates to a composition for decomposing fat including the *Leuconostoc holzapfelii* strain Ceb-kc-003 (KCCM11830P), a culture solution thereof, a concentrate thereof or a dried product thereof. A further embodiment of the present invention relates to a method for preventing or treating hyperlipidemia including administering to a subject in need of such prevention or treatment a therapeutically or prophylactically effective amount of the *Leuconostoc holzapfelii* strain Ceb-kc-003 (KCCM11830P), a culture solution thereof, a concentrate thereof or a dried product thereof. The therapeutically or prophylactically effective amount is the same as that described above.

### [Advantageous Effects]

The *Leuconostoc holzapfelii* strain Ceb-kc-003 (KCCM11830P) and the composition including the strain are effective in preventing hair loss, promoting hair growth or improving sexual function.

In addition, the *Leuconostoc holzapfelii* strain Ceb-kc-003 (KCCM11830P) and the composition including the strain can be used safely without causing side effects to prevent hair loss, promote hair growth or improve sexual function.

### [Description of Drawings]

FIG. 1 shows photographs comparing scalp hair before and ∼3.5 months after taking *Leuconostoc holzapfelii* Ceb-kc-003 (KCCM11830P).
FIG. 2 shows photographs comparing scalp hair before and ∼2.5 and ∼3.5 months after taking *Leuconostoc holzapfelii* Ceb-kc-003 (KCCM11830P).
FIG. 3 shows photographs comparing scalp hair before and ∼1.7 months after taking *Leuconostoc holzapfelii* Ceb-kc-003 (KCCM11830P).
FIG. 4 shows photographs comparing scalp hair before and ∼3 months after taking *Leuconostoc holzapfelii* Ceb-kc-003 (KCCM11830P).
FIG. 5 is a histogram comparing the effects of *Leuconostoc holzapfelii* Ceb-kc-003 (KCCM11830P) and another lactic acid bacterial strain on cholesterol levels.
FIG. 6 is a histogram comparing the effects of *Leuconostoc holzapfelii* Ceb-kc-003 (KCCM11830P) and another lactic acid bacterial strain on fat decomposition.
FIG. 7 is histogram comparing the effects of *Leuconostoc holzapfelii* Ceb-kc-003 (KCCM11830P) and another lactic acid bacterial strain on NO production.

### [Mode for Invention]

The present invention will be described in more detail with reference to the following examples. However, these examples are provided for illustrative purposes only and the present invention is not limited thereto.

### Example 1: Isolation and identification of Leuconostoc holzapfelii Ceb-kc-003 (KCCM11830P)

### (1) Sampling and strain isolation

A sample was taken from kimchi, a traditional Korean fermented food, diluted stepwise, plated on a BHI solid medium (Difco, USA) supplemented with 3% sodium chloride, and cultured at 37°C for 24 h. A dominant strain was isolated from the sample. Colonies were selected and passaged three times in fresh media. The pure cultured bacterial strain was placed in a medium supplemented with 20% glycerol and stored at ≤-70°C.

### (2) Investigation of morphological and taxological properties

The isolated strain was identified. To this end, the strain was primarily morphologically and biochemically investigated. The strain was found to be morphologically Gram-positive by Gram staining. The taxological properties of the strain were analyzed by 16s rRNA partial sequencing. As a result, the isolated strain was found to have the sequence set forth in SEQ ID NO: 1 and a homology of 99% with *Leuconostoc holzapfelii.*

The newly isolated strain *Leuconostoc holzapfelii* Ceb-kc-003 was deposited with the Korean Culture Center of Microorganisms (120-861, Hongje-2ga-Gil, Seodaemun-Ku, Seoul, Korea) on April 11, 2016 and assigned accession number KCCM11830P.

The strain was freeze-dried and powdered.

### (3) Preparation of culture solution of the strain

A culture solution of the *Leuconostoc holzapfelii* strain Ceb-kc-003 was prepared by the following procedure:

2 kg of dextrose, 1.5 kg of whole milk powder, and 0.05 kg of an yeast extract were added to 100 liters of purified water. The mixture was sterilized in an autoclave at 121°C for 15-30 min and cooled to around 35°C. Thereafter, the sterile mixture was inoculated with 0.2-0.4 liters of the *Leuconostoc holzapfelii* strain Ceb-kc-003, followed by culture at around 35°C for 2-3 days.

### Example 2: Preventive effect of the Leuconostoc holzapfelii strain Ceb-kc-003 (KCCM11830P) on hair loss

The culture solution of the *Leuconostoc holzapfelii* strain Ceb-kc-003 (KCCM11830P) at a concentration of 1 × 10⁶ to 1 × 10⁸ CFU/ml was administered to a total of 5 men aged 40-85 years and one woman who suffered from alopecia twice daily (50-100 ml each time) over 1-4 months.

The numbers of hairs lost were counted before and 10, 20, and 30 days after taking the strain. The patients were instructed to wash their hair before hair counting.

The experimental results are shown in Table 1.

**Table 11**

| **Subject Time** | **Man at age 45** | **Man at age 52** | **Woman at age 38** | **Men at age 57** |
|---|---|---|---|---|
| Before taking | 60 hairs lost | 43 hairs lost | 74 hairs lost | 23 hairs lost |
| 10 days after taking | 40 hairs lost | 32 hairs lost | 55 hairs lost | 10 hairs lost |
| 20 days after taking | 22 hairs lost | 15 hairs lost | 23 hairs lost | 4 hairs lost |
| 30 days after taking | 12 hairs lost | 5 hairs lost | 8 hairs lost | 2 hairs lost |

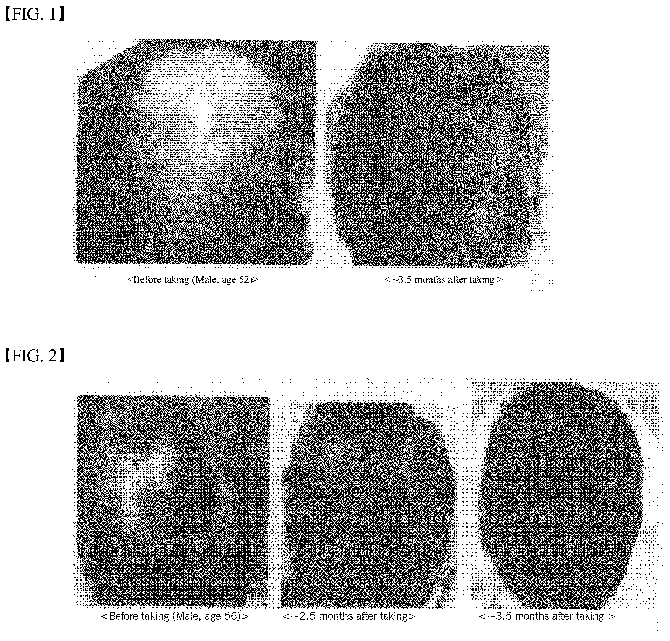

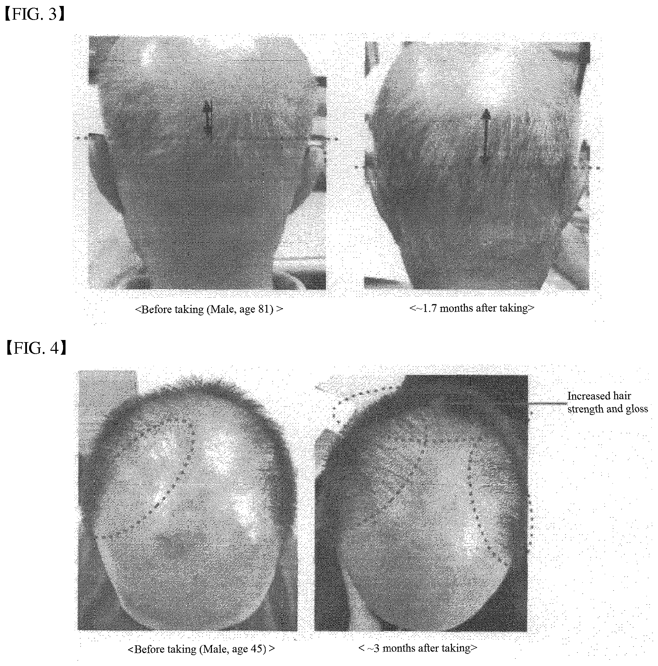

The hairs of the male patients before and at the predetermined time points after administration of the strain were photographed to measure their thicknesses, numbers, and glosses. Some of the results are shown in FIGs. 1-4.

These results indicate that the *Leuconostoc holzapfelii* strain Ceb-kc-003 (KCCM11830P) is effective in preventing hair loss and markedly improving the thickness, number, and gloss of hairs. Therefore, it can be conclude that the *Leuconostoc holzapfelii* strain Ceb-kc-003 (KCCM11830P) is effective in treating hair loss.

### Example 3: Effect of the Leuconostoc holzapfelii strain Ceb-kc-003 (KCCM11830P) on reducing cholesterol levels

The effect of the *Leuconostoc holzapfelii* strain Ceb-kc-003 (KCCM11830P) on reducing cholesterol levels was examined. To this end, solutions of the powder of the *Leuconostoc holzapfelii* strain Ceb-kc-003 (KCCM11830P) (1-5 mg/ml) and solutions of a powder of *Lactobacillus plantarum* (1-5 mg/ml) as controls were used.

### <Ability to remove cholesterol>

The ability of the strains to remove cholesterol was specifically measured by the following procedure. 30 ml of an MRS culture ground was added to a 50 ml Falcon tube, inoculated with the test strain (glycerol stock) at a density of 1 × 10⁷ counts/ml, and cultured at 30°C for 24 h. 300 µl of the culture solution was added to 30 ml of an MRS culture ground containing 350 mg/L cholesterol (Wako Pure Chemical) and 0.2% bile acid (w/v). After culture at 37°C for 24 h, the culture solution was subjected to centrifugal separation at 8,000 rpm and 4°C for 10 min. The cholesterol level of the supernatant was measured using Determiner FC (Kyowa Medex Co., Ltd.). The level (%) of cholesterol removed from the culture ground was calculated by subtracting the remaining cholesterol level from the initial cholesterol level of the cholesterol culture ground.

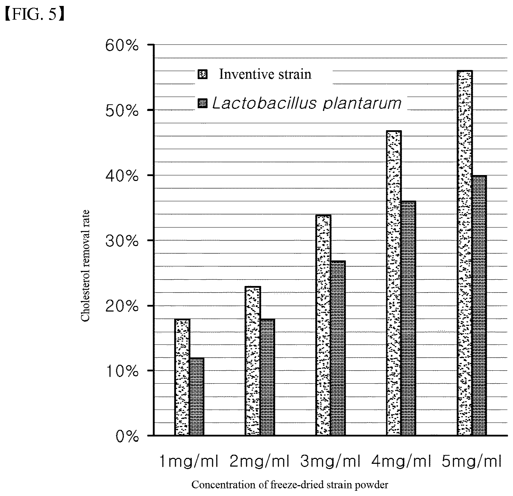

The results are shown in FIG. 5. This experiment was conducted in triplicate and the data were averaged.

From these results, it can be concluded that the powder of the *Leuconostoc holzapfelii* strain Ceb-kc-003 (KCCM11830P) is significantly effective in reducing cholesterol levels in proportion to the strain concentration compared to the other lactic acid bacterial species *Lactobacillus plantarum.*

### Example 4: Effect of the Leuconostoc holzapfelii strain Ceb-kc-003 (KCCM11830P) on fat decomposition

In this example, the effect of the *Leuconostoc holzapfelii* strain Ceb-kc-003 (KCCM11830P) on fat decomposition was examined. To this end, 1000 cc of pork fat was treated with 20-100 ml of culture solutions of the strain, followed by shaking at 37°C for 24 h. This procedure was repeated for *Lactobacillus plantarum* as a control.

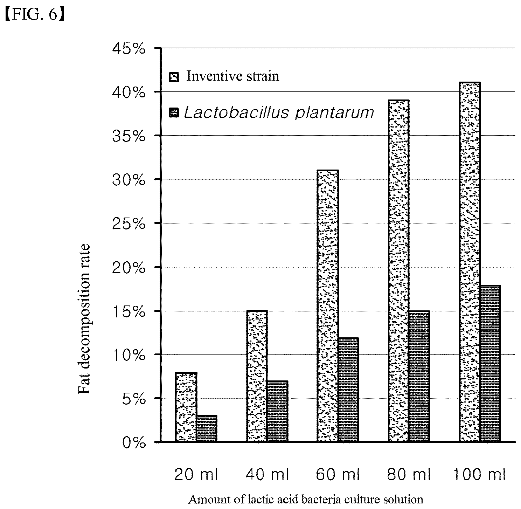

The results are shown in FIG. 6.

From these results, it can be concluded that the powder of the *Leuconostoc holzapfelii* strain Ceb-kc-003 is significantly effective in fat decomposition in proportion to the strain concentration compared to the other lactic acid bacterial species *Lactobacillus plantarum.*

### Example 5: Effects of the Leuconostoc holzapfelii strain Ceb-kc-003 (KCCM11830P) on improving sexual function and preventing sexual dysfunction

Th powders of the *Leuconostoc holzapfelii* strain Ceb-kc-003 (KCCM11830P) at concentrations of 1 × 10⁶ to 1 × 10⁸ CFU/ml were administered orally to a total of 10 male smokers or non-smokers, aged 45-70 years, once or twice (50 ml to 100 ml each time) daily over a total of 3 months. The frequency of erections in the early morning, erectility, and duration of erection were self-evaluated before and at 1-month intervals after administration of the strain.

4 out of the 10 men answered questionnaires for 1-3 months after taking. The results are shown in Table 2.

**[Table 2]**

| Data on effects of the strain on improving in sexual function | | | | |
|---|---|---|---|---|
| **Subject Time** | **Male non-smoker at age 45** | **Male non-smoker at age 54** | **Male smoker at age 63** | **Male smoker at age 57** |
| Before taking | Good erectility | Good erectility | Impotence | Poor erectility |
| 1 month after taking | Increased frequency of erections in the early morning | Good erectility | Impotence | Poor erectility |
| 2 months after taking | Increased frequency of erections in the early morning | Increased duration of erection | Erection in the early morning | Increased frequency of erections in the early morning |
| 3 months after taking | Increased duration of erection | Increased frequency of erections | Increased frequency of erections in the early morning | Increased duration of erection |

The above results indicate that continuous taking of the powder of the *Leuconostoc holzapfelii* strain Ceb-kc-003 is effective in preventing and treating sexual dysfunction.

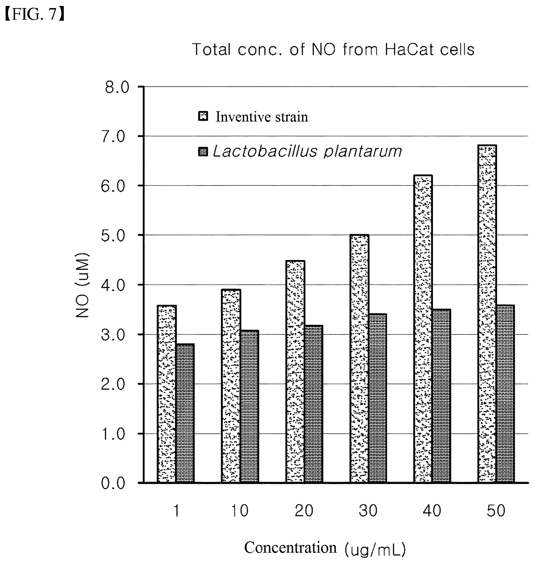

In addition, the concentrations of NO released from Hacat cells for 24 h were evaluated. NO is helpful in improving erectility due to its ability to increase blood circulation and dilate capillaries. The concentrations of NO produced after taking the novel strain of the present invention were compared with those after taking *Lactobacillus plantarum.* This experiment was conducted in triplicate and the data were averaged. The results are shown in FIG. 7.

The effects of culture solutions of *Leuconostoc mesenteroides* (KCCM11827P) and *Lactobacillus sakei* (KCCM11841P) strains together with the *Leuconostoc holzapfelii* strain Ceb-kc-003 (KCCM11830P) on the prevention and the treatment of hair loss and the improvement of sexual function were evaluated in the same manner as described above. As a result, better results were obtained compared to the single use of the *Leuconostoc holzapfelii* strain Ceb-kc-003.

## Claims

1. A *Leuconostoc holzapfelii* strain Ceb-kc-003 (KCCM11830P).

2. The strain according to claim 1 for use in preventing hair loss, promoting hair growth, ameliorating erectile dysfunction, lowering cholesterol levels or decomposing fat.

3. A culture solution, concentrate or dried product comprising *Leuconostoc holzapfelii* Ceb-kc-003 (KCCM11830P).

4. A food composition comprising a *Leuconostoc holzapfelii* strain Ceb-kc-003 (KCCM11830P) or a culture solution, concentrate or dried product comprising said strain.

5. The food composition according to claim 4, wherein the food composition is effective in preventing hair loss or promoting hair growth.

6. The food composition according to claim 4, wherein the food composition is effective in ameliorating erectile dysfunction.

7. The food composition according to claim 4, wherein :
- the daily dose of the *Leuconostoc holzapfelii* Ceb-kc-003 (KCCM11830P) strain is from 1.5 × 10⁵ to 5 × 10¹⁰ CFU; or
- the *Leuconostoc holzapfelii* Ceb-kc-003 (KCCM11830P) strain at a concentration of 5 × 10⁴ to 5 × 10⁸ CFU/ml is administered 1 to 4 times (30 ml to 100 ml each time) daily.

8. The food composition according to any one of claims 4 to 7, further comprising a strain belonging to the genus *Lactobacillus, Lactococcus, Propionibacterium, Enterocuccus* or *Bifidobacterium.*

9. A pharmaceutical composition comprising a *Leuconostoc holzapfelii* strain Ceb-kc-003 (KCCM11830P) or a culture solution, concentrate or dried product comprising said strain for use in preventing hair loss or promoting hair growth.

10. A pharmaceutical composition comprising a *Leuconostoc holzapfelii* strain Ceb-kc-003 (KCCM11830P) or a culture solution, concentrate or dried product comprising said strain for use in ameliorating erectile dysfunction.

11. The pharmaceutical composition for use according to claim 10, wherein the *Leuconostoc holzapfelii* strain Ceb-kc-003 (KCCM11830P) is administered in an amount of 0.0001 mg/kg to 10 g/kg daily.

12. The pharmaceutical composition for use according to claim 9, wherein the *Leuconostoc holzapfelii* strain Ceb-kc-003 (KCCM11830P) at a concentration of 5 × 10⁴ to 5 × 10⁸ CFU/ml is administered 1 to 4 times (30 ml to 100 ml each time) daily.

13. The pharmaceutical composition for use according to any one of claims 9 to 12, further comprising :
- a pharmaceutically acceptable carrier;
- a strain belonging to the genus *Lactobacillus, Lactococcus, Propionibacterium, Enterocuccus* or *Bifidobacterium* ; or
- a *Leuconostoc mesenteroides* strain (KCCM11827P), a *Lactobacillus sakei* strain (KCCM11841P), a culture solution thereof, a concentrate thereof, a dried product thereof or a mixture thereof.

14. The pharmaceutical composition for use according to any one of claims 9 to 12, wherein the *Leuconostoc holzapfelii* strain Ceb-kc-003 (KCCM11830P) is isolated from kimchi.

15. The pharmaceutical composition for use according to any one of claims 9 to 12, wherein the *Leuconostoc holzapfelii* strain Ceb-kc-003 (KCCM11830P) is present in an amount of 0.05 wt% to 80 wt%, based on the weight of the pharmaceutical composition.

## Patentansprüche

1. *Leuconostoc-holzapfelii-Stamm* Ceb-kc-003 (KCCM11830P).

2. Stamm nach Anspruch 1 zur Verwendung bei der Vorbeugung von Haarausfall, der Förderung von Haarwuchs, der Linderung von erektiler Dysfunktion, der Senkung von Cholesterinwerten oder dem Abbau von Fett.

3. Nährlösung, Konzentrat oder getrocknetes Produkt, umfassend *Leuconostoc holzapfelii* Ceb-kc-003 (KCCM11830P).

4. Nahrungsmittelzusammensetzung, umfassend einen *Leuconostoc-holzapfelii-*Stamm Ceb-kc-003 (KCCM11830P) oder eine Nährlösung, ein Konzentrat oder ein getrocknetes Produkt, welche diesen Stamm umfassen.

5. Nahrungsmittelzusammensetzung nach Anspruch 4, wobei die Nahrungsmittelzusammensetzung wirksam bei der Vorbeugung von Haarausfall oder der Förderung von Haarwuchs ist.

6. Nahrungsmittelzusammensetzung nach Anspruch 4, wobei die Nahrungsmittelzusammensetzung wirksam bei der Linderung von erektiler Dysfunktion ist.

7. Nahrungsmittelzusammensetzung nach Anspruch 4, wobei:
- die tägliche Dosis des *Leuconostoc-holzapfelii-Stamms* Ceb-kc-003 (KCCM11830P) von 1,5 × 10⁵ bis 5 × 10¹⁰ CFU beträgt oder
- der *Leuconostoc-holzapfelii*-Stamm Ceb-kc-003 (KCCM11830P) täglich ein bis vier Mal (jeweils 30 ml bis 100 ml) in einer Konzentration von 5 × 104 bis 5 × 10⁸ CFU/ml verabreicht wird.

8. Nahrungsmittelzusammensetzung nach einem der Ansprüche 4 bis 7, ferner umfassend einen Stamm, der der Gattung *Lactobacillus, Lactococcus, Propionibacterium, Enterococcus* oder *Bifidobacterium* angehört.

9. Pharmazeutische Zusammensetzung, umfassend einen *Leuconostoc-holzapfelii-*Stamm Ceb-kc-003 (KCCM11830P) oder eine Nährlösung, ein Konzentrat oder ein getrocknetes Produkt, welche diesen Stamm umfassen, zur Verwendung bei der Vorbeugung von Haarausfall oder der Förderung von Haarwuchs.

10. Pharmazeutische Zusammensetzung, umfassend einen *Leuconostoc-holzapfelii-*Stamm Ceb-kc-003 (KCCM11830P) oder eine Nährlösung, ein Konzentrat oder ein getrocknetes Produkt, welche diesen Stamm umfassen, zur Verwendung bei der Linderung von erektiler Dysfunktion.

11. Pharmazeutische Zusammensetzung zur Verwendung nach Anspruch 10, wobei der *Leuconostoc-holzapfelii-Stamm* Ceb-kc-003 (KCCM11830P) in einer täglichen Menge von 0,0001 mg/kg bis 10 g/kg verabreicht wird.

12. Pharmazeutische Zusammensetzung zur Verwendung nach Anspruch 9, wobei der *Leuconostoc-holzapfelii-Stamm* Ceb-kc-003 (KCCM11830P) täglich ein bis vier Mal (jeweils 30 ml bis 100 ml) in einer Konzentration von 5 × 104 bis 5 × 10⁸ CFU/ml verabreicht wird.

13. Pharmazeutische Zusammensetzung zur Verwendung nach einem der Ansprüche 9 bis 12, ferner umfassend:
- einen pharmazeutisch verträglichen Träger,
- einen Stamm, der der Gattung *Lactobacillus, Lactococcus, Propionibacterium, Enterococcus* oder *Bifidobacterium* angehört, oder
- einen *Leuconostoc-mesenteroides-Stamm* (KCCM11827P), einen *Lactobacillus-sakei-Stamm* (KCCM11841P), eine Nährlösung davon, ein Konzentrat davon, ein getrocknetes Produkt davon oder eine Mischung davon.

14. Pharmazeutische Zusammensetzung zur Verwendung nach einem der Ansprüche 9 bis 12, wobei der *Leuconostoc-holzapfelii-Stamm* Ceb-kc-003 (KCCM11830P) aus Kimchi isoliert ist.

15. Pharmazeutische Zusammensetzung zur Verwendung nach einem der Ansprüche 9 bis 12, wobei der *Leuconostoc-holzapfelii-Stamm* Ceb-kc-003 (KCCM11830P) bezogen auf das Gewicht der pharmazeutischen Zusammensetzung in einer Menge von 0,05 Gew.-% bis 80 Gew.-% vorliegt.

## Revendications

1. Souche de *Leuconostoc holzapfelii* Ceb-kc-003 (KCCM 11830P).

2. Souche selon la revendication 1 pour une utilisation dans la prévention de la perte des cheveux, la promotion de la pousse des cheveux, l'amélioration de la dysfonction érectile, la diminution des teneurs en cholestérol ou la décomposition de graisse.

3. Solution de culture, concentré ou produit séché comprenant *Leuconostoc holzapfelii* Ceb-kc-003 (KCCM11830P).

4. Composition alimentaire comprenant une souche de *Leuconostoc holzapfelii* Ceb-kc-003 (KCCM11830P) ou une solution de culture, un concentré ou un produit séché comprenant ladite souche.

5. Composition alimentaire selon la revendication 4, dans laquelle la composition alimentaire est efficace dans la prévention de la chute des cheveux ou la promotion de la pousse des cheveux.

6. Composition alimentaire selon la revendication 4, dans laquelle la composition alimentaire est efficace pour améliorer une dysfonction érectile.

7. Composition alimentaire selon la revendication 4, dans laquelle :
- la dose quotidienne de la souche de *Leuconostoc holzapfelii* Ceb-kc-003 (KCCM11830P) est de 1,5 x 10⁵ à 5 x 10¹⁰ CFU ; ou
- la souche de *Leuconostoc holzapfelii* Ceb-kc-003 (KCCM11830P) à une concentration de 5 x 10⁴ à 5 x 10⁸ CFU/ml est administrée 1 à 4 fois (30 ml à 100 ml chaque fois) quotidiennement.

8. Composition alimentaire selon l'une quelconque des revendications 4 à 7, comprenant de plus une souche appartenant au genre *Lactobacillus, Lactococcus, Propionibacterium, Enterocuccus* ou *Bifidobacterium.*

9. Composition pharmaceutique comprenant une souche de *Leuconostoc holzapfelii* Ceb-kc-003 (KCCM11830P) ou une solution de culture, un concentré ou un produit séché comprenant ladite souche pour une utilisation dans la prévention de la chute des cheveux ou la promotion de la pousse des cheveux.

10. Composition pharmaceutique comprenant une souche de *Leuconostoc holzapfelii* Ceb-kc-003 (KCCM11830P) ou une solution de culture, un concentré ou un produit séché comprenant ladite souche pour une utilisation dans l'amélioration d'une dysfonction érectile.

11. Composition pharmaceutique pour une utilisation selon la revendication 10, dans laquelle la souche de *Leuconostoc holzapfelii* Ceb-kc-003 (KCCM11830P) est administrée dans une quantité de 0,0001 mg/kg à 10 g/kg quotidiennement.

12. Composition pharmaceutique pour une utilisation selon la revendication 9, dans laquelle la souche de *Leuconostoc holzapfelii* Ceb-kc-003 (KCCM11830P) à une concentration de 5 x 10⁴ à 5 x 10⁸ CFU/ml est administrée 1 à 4 fois (30 ml à 100 ml chaque fois) quotidiennement.

13. Composition pharmaceutique pour une utilisation selon l'une quelconque des revendications 9 à 12, comprenant de plus :
- un support pharmaceutiquement acceptable ;
- une souche appartenant au genre *Lactobacillus, Lactococcus, Propionibacterium, Enterocuccus* ou *Bifidobacterium* ; ou
- une souche de *Leuconostoc mesenteroides* (KCCM11827P), une souche de *Lactobacillus sakei* (KCCM11841P), une solution de culture de celles-ci, un concentré de celles-ci, un produit séché de celles-ci ou un mélange de ceux-ci.

14. Composition pharmaceutique pour une utilisation selon l'une quelconque des revendications 9 à 12, dans laquelle la souche de *Leuconostoc holzapfelii* Ceb-kc-003 (KCCM11830P) est isolée à partir de kimchi.

15. Composition pharmaceutique pour une utilisation selon l'une quelconque des revendications 9 à 12, dans laquelle la souche de *Leuconostoc holzapfelii* Ceb-kc-003 (KCCM11830P) est présente dans une quantité de 0,05 % en masse à 80 % en masse, sur la base de la masse de la composition pharmaceutique.
